# EUROPÄISCHE PATENTSCHRIFT

(11) **EP 1 289 423 B1**
(45) Veröffentlichungstag und Bekanntmachung des Hinweises auf die Patenterteilung: **02.08.2006**
(21) Anmeldenummer: 01955189.4
(22) Anmeldetag: 05.06.2001
(51) Int. Cl.: A61B 6/04, A61G 13/02

(54) **OPERATIONS-DIAGNOSE-EINRICHTUNG MIT EINER LAGERUNGSVORRICHTUNG FÜR EIN BEHANDLUNGS- UND/ODER UNTERSUCHUNGSOBJEKT**
OPERATION-DIAGNOSTIC UNIT COMPRISING A SUPPORTING DEVICE FOR AN OBJECT TO BE TREATED AND/OR EXAMINED
UNITE D'OPERATION/DE DIAGNOSTIC COMPORTANT UN DISPOSITIF POUR INSTALLER UN OBJET A TRAITER ET/OU A EXAMINER

(30) Priorität: 15.06.2000 DE 10029429
(43) Veröffentlichungstag der Anmeldung: 12.03.2003
(73) Patentinhaber: SIEMENS AKTIENGESELLSCHAFT, 80333 München (DE)
(72) Erfinder: BARDE, Karlheinz, 95469 Speichersdorf (DE); HEINL, Dieter, 92681 Erbendorf (DE); SCHÖCKLMANN, Thomas, 95478 Kemnath (DE); WALDBACH, Kerstin, 07778 Porstendorf (DE)
(86) Internationale Anmeldenummer: PCT/DE2001/002102
(87) Internationale Veröffentlichungsnummer: WO 2001/095807

(56) Entgegenhaltungen:
- EP-A- 0 923 922
- DE-A- 19 920 008
- JP-A- 11 313 900
- US-A- 3 868 103

## Beschreibung

Die Erfindung bezieht sich auf eine Operations-Diagnose-Einrichtung mit einer Lagerungsvorrichtung für ein Behandlungs- und/oder Untersuchungsobjekt, die eine an einem Sockel verstellbare Lagerungsplatte für das Behandlungs- und/oder Untersuchungsobjekt, aufweist, wobei die Lagerungsplatte höhenverstellbar und um drei Raumachsen verstellbar gelagert ist, und in Längs- und/oder Querrichtung durch eine Antriebsvorrichtung verschiebbar ist, wobei die Lagerungsplatte um eine Längsachse (Kantachse) und um eine Querachse (Kippachse) schwenkbar gelagert ist

Für angiographische Untersuchungen sind Lagerungsvorrichtungen für ein Untersuchungsobjekt bekannt, die eine Lagerungsplatte aufweisen, die an einem Sockel entlang ihrer Längs- und Querachse sowie höhenverstellbar gelagert sind. Die Lagerungsplatte ist zumindest in einem Bereich strahlungstransparent, so dass in Verbindung mit einer Aufnahmeeinheit aus Strahlensender und Strahlenempfänger Durchstrahlungsaufnahmen des Untersuchungsobjektes erstellbar sind. Der Strahlensender und der Strahlenempfänger können hierzu beispielsweise an den Enden eines C-Bogens einander gegenüberliegend lagern, der an einer Halterung entlang seines Umfanges sowie beispielsweise an Decken- oder Bodenschienen verstellbar gelagert ist.

Soll ein Untersuchungsobjekt nach dem Erstellen von beispielsweise Röntgenaufnahmen am offenen Gefäß behandelt werden, wie das bei angiographischen Untersuchungen häufig der Fall ist, so muss das Untersuchungsobjekt von der Angiographieanlage in den Operationssaal befördert und dort auf dem Operationstisch gelagert werden. Angiographische Arbeitsplätze sind so ausgeführt, dass sie die röntgendiagnostische und auch die Behandlung mittels eines Katheters erlauben, jedoch sind sie nicht geeignet, beispielsweise Operationen am offenen Gefäß durchzuführen, weil hierzu die hygienischen Anforderungen und die Geräteausstattung nicht ausreicht.

Aus der JP 11 313900 A, die der nachveröffentlichten US 6 094 760 A entspricht, ist bereits eine Einrichtung der eingangs genannten Art bekannt geworden, die allerdings nur zur Strahlenbehandlung, nicht aber zur Strahlenuntersuchung und auch nicht zu Operationszwecken gedacht und geeignet ist. Zur Verschiebung der Lagerplatte in Längs- und Querrichtung wird dort der gesamte Sockel im Raum verfahren, was sowohl für einen Operationstisch ungeeignet ist, als auch den Einsatz einer Strahlenuntersuchungseinrichtung in Verbindung mit einer solchen Operationsdiagnoseeinrichtung verhindern oder doch sehr erschweren würde.

Aufgabe der Erfindung ist es daher, Möglichkeiten zur Verfügung zu stellen, durch die sowohl eine gute Diagnose als auch eine Behandlung, insbesondere Operationen, möglich ist, ohne dass hierbei das Behandlungs- oder Untersuchungsobjekt einer Umlagerung bedarf.

Zur Lösung dieser Aufgabe ist erfindungsgemäß eine Operations-Diagnose-Einrichtung gemäß Anspruch 1 vorgesehen.

Eine solche Operations-Diagnose-Einrichtung ermöglicht sowohl die Verstellung der Lagerungsplatte wie sie bei einer Operations erforderlich ist, als auch aufgrund der Strahlentransparenz in Verbindung mit der Verstellmöglichkeit eines Angiographie-Arbeitsplates eine Strahldiagnose, insbesondere angiographische Untersuchungen, wobei eine Umlagerung des Behandlungs- und/oder Untersuchungsobjektes nicht erforderlich ist.

Von besonderer Bedeutung ist dabei, dass eine Verschwenkung um eine parallel zur Kippachse verlaufende virtuelle Schwenkachse des Isozentrums des Behandlungs- und/oder Untersuchungsobjekts, also beispielsweise eines Patienten, erfolgt und gleichzeitig eine vorgebbare Körperregion im Isozentrum angeordnet ist und damit an Ort und Stelle verbleibt. Beispielsweise kann es sich hierbei um den Kopf, das Herz oder ein sonstiges Organ handeln, speziell ein Organ, an dem operative Eingriffe erfolgen sollen.

Der erfindungsgemäße Aufbau ermöglicht eine Vielzahl von unterschiedlichen Bewegungsmöglichkeiten an einem Tisch:
- Rotation (optional manuell/motorisch)
- Hubbewegung
- Kantbewegung
- Kippbewegung (optional ± 15° oder - 15°/+ 90°)
- kombinierte Kipp-/Kantbewegung
- Längsbewegung (optional manuell/motorisch)
- Querbewegung (optional manuell/motorisch)
- kombinierte Längs-/Querbewegunga (optional manuell/motorisch)
- kombinierte Hub-/Kipp-/Längsbewegung
- kombinierte Hub-/Kant-/Querbewegung
- Längs- oder Querbewegung (in jeder Kipp- oder Kantposition möglich) Dabei liegt es schließlich auch noch im Rahmen der Erfindung, die zumindest bereichfreie strahlentransparente Lagerungsplatte austauschbar zu gestalten, so dass wahlweise eine Angiographie-Lagerungsplatte, eine OP-Lagerungsplatte mit einer oder mehreren Knickstellen oder auch eine CT-Lagerungsplatte (gewölbt) angebracht werden können.

Zu diesem Zweck ist weiter vorgesehen, dass eine Aufnahmeeinheit aus Strahlensender und Strahlenempfänger vorhanden ist, die von einer Parkposition, in der ein freier Zugang zur Lagerungsvorrichtung möglich ist, in eine Aufnahmeposition zur Erstellung von Strahlenaufnahmen bringbar ist. Beispielsweise eignet sich hierfür ein sogenannter C-Bogen, an dessen beiden Enden die Aufnahmeeinheit lagert, wobei der C-Bogen selbst wiederum in an sich bekannter Weise entweder verstellbar an der Decke eines Raums oder an einem Bodensockel verstellbar gelagert sein kann.

Vorzugsweise umfasst die Operations-Diagnose-Einrichtung einen Satz austauschbarer, unterschiedlicher Lagerungsplatten. Alle Lagerungsplatten des Satzes sind an dem Sockel anbringbar.

Der Satz umfasst vorzugsweise eine Angiographie-Lagerungsplatte, eine OP-Lagerungsplatte und/oder eine Computertomographie(CT)-Lagerungsplatte.

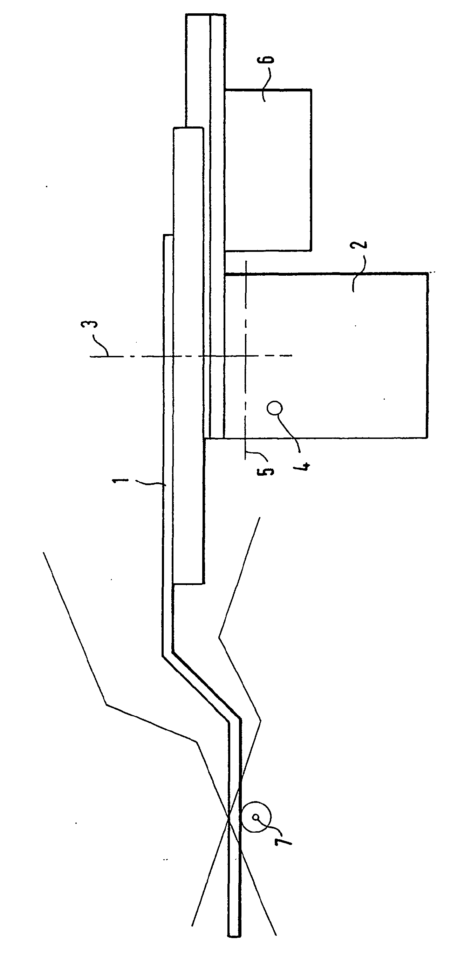

Weitere Vorteile, Merkmale und Einzelheiten der Erfindung ergeben sich aus der nachfolgenden Beschreibung eines Ausführungsbeispiels sowie anhand der Zeichnung, die schematisch eine erfindungsgemäße Lagerungsvorrichtung in Seitenansicht zeigt.

Diese Lagerungsvorrichtung umfasst eine knickbare, teilweise strahlentransparent, ausgebildete Lagerungsplatte 1, die verstellbar an einem Sockel 2 angeordnet ist, der auch die wesentlichen Antriebsaggregate zur Verstellung der Position der Lagerungsplatte aufnehmen kann. Neben einer Höhenverstellung in Richtung der Achse 3 und einer Verschwenkbarkeit der Lagerungsplatte 1 um diese vertikale Achse 3 kann die Lagerungsplatte 1 auch um eine Querachse (Kippachse 4) und um eine dazu senkrechte Längsachse (Kantachse 5) verschwenkt werden, wobei alle diese Bewegungen vorzugsweise motorisch durch elektrische oder hydraulische Antriebe erfolgen können. Bevorzugt sollen die Antriebe für die Drehbewegungen auch abschaltbar sein, so dass die entsprechenden Bewegungen von Hand erfolgen können.

Darüber hinaus - und dies ist für die vorliegende Erfindung von besonderer Bedeutung - ist die Lagerungsplatte 1 in Längsrichtung und in Querrichtung verschiebbar, wobei diese Verschiebung zum einen mit Hilfe einer nur schematisch angedeuteten Antriebsvorrichtung 6 erfolgen kann. Diese Antriebsvorrichtung soll in der Weise mit der Lagerungsplatte 1 gekoppelt sein, dass die Antriebsvorrichtung 6 wahlweise völlig abgeschaltet werden kann, so dass dann eine schwimmende Lagerung der Lagerungsplatte gegeben ist, die eine einfache Feinverstellbarkeit und Einjustierbarkeit des Behandlungs- und/oder Untersuchungsobjekts bei horizontaler Lagerungsplatte ermöglicht. Immer dann, wenn die Lagerungsplatte gekippt oder verkantet ist, soll, vorzugsweise automatisch, die Antriebsvorrichtung 6 angekoppelt sein, um auf diese Art und Weise die Arretierung der Lagerungsplatte in der jeweiligen Position ohne die Notwendigkeit gesonderter Arretiereinrichtungen zu gewährleisten.

Die Antriebsvorrichtung für beispielsweise die Verschwenkung um die Kippachse 4 kann in der zentralen Steuerungsvorrichtung derart über ein Programm mit dem Hubantrieb zur Höhenverstellung in Richtung der vertikalen Achse 3 und der Antriebsvorrichtung 6 gekoppelt sein, das über dieses Programm die Lagerungsplatte um ein vorgebbares virtuelles Isozentrum 7 schwenkt, dessen Position ebenfalls vorgebbar sein kann.

Das Zuschalten der Antriebsvorrichtung 6, also die Verbindung der Lagerungsplatte 1 mit dieser Antriebsvorrichtung (die dabei natürlich nicht tatsächlich aktiviert sein muss, die aber durch das Zuschalten ohne Freilauf als Arretierung wirkt) ist stets erforderlich, ehe eine Verschwenkung um eine der Drehachsen 3, 4 und 5 erfolgt, d. h. die schwimmende Lagerung der Lagerungsplatte 1 kann nur dann angewendet werden, wenn die Lagerungsplatte horizontal steht und keine Verkippung vorgenommen werden soll.

## Patentansprüche

1. Operations-Diagnose-Einrichtung mit einer Lagerungsvorrichtung für ein Behandlungs- und/oder Untersuchungsobjekt, die eine an einem Sockel verstellbare Lagerungsplatte für das Behandlungs- und/oder Untersuchungsobjekt, aufweist, wobei die Lagerungsplatte (1) höhenverstellbar und um drei Raumachsen (3, 4, 5) verstellbar gelagert ist, und in Längs- und/oder Querrichtung durch eine Antriebsvorrichtung (6) verschiebbar ist, wobei die Lagerungsplatte (1) um eine Längsachse (5) und um eine Querachse (4) schwenkbar gelagert ist, **dadurch gekennzeichnet, dass** die Lagerungsplatte zumindest in einem Bereich zur Erstellung von Durchgtrahlungsaufnahmen strahlungstransparent ist; und dass sie mit der Antriebsvorrichtung derart gekoppelt ist, dass die Antriebsvorrichtung wahlweise bei horizontal stehender Lagerungsplatte völlig abschaltbar ist, sodass dann eine schwimmende Lagerung der Lagerungsplatte gegeben ist, wobei bei gekippter oder verkanteter Lagerungsplatte die Antriebsvorrichtung zur Arretierung automatisch angekoppelt ist und wobei die Antriebsvorrichtung für die Verschwenkung um die Querachse (4) und/oder die Längsachse (5) derart mit dem Hubantrieb zur Höhenverstellung und der Antriebsvorrichtung (6) zur Verschiebung der Lagerungsplatte (1) gekoppelt ist, dass die Lagerungsplatte (1) um ein vorgebbares Isozentrum (7) schwenkbar ist und dass eine Aufnahmeeinheit aus Strahlensender und Strahlenempfänger von einer Parkposition, in der ein freier Zugang zur Lagerungsvorrichtung möglich ist, in eine Aufnahmeposition zur Erstellung von Strahlenaufnahmen bringbar ist.

2. Operations-Diagnose-Einrichtung nach Anspruch 1, **dadurch gekennzeichnet, dass** Bewegungen der Lagerungsplatte (1) um eine der Drehachsen (3, 4, 5) nur bei zugeschalteter Antriebsvorrichtung (6) für die Längs- und Querverschiebung möglich sind.

3. Operations-Diagnose-Einrichtung nach Anspruch 1 oder 2, **dadurch gekennzeichnet, dass** die Lagerungsplatte (1) austauschbar angeordnet ist.

4. Operations-Diagnose-Einrichtung nach Anspruch 3, **dadurch gekennzeichnet, dass** die Lagerungsplatte (1) wahlweise als Angiographie-Lagerungsplatte, OP-Lagerungsplatte mit einer oder mehreren Knickstellen oder als CT-Lagerungsplatte ausgebildet ist.

5. Operations-Diagnose-Einrichtung nach Anspruch 3 oder 4 mit einem Satz austauschbarer unterschiedlicher Lagerungsplatten (1).

6. Operations-Diagnose-Einrichtung nach Anspruch 5, wobei der Satz eine Angiographie-Lagerungsplatte, eine OP-Lagerungsplatte und/oder eine CT-Lagerungsplatte umfasst.

## Claims

1. Operation-diagnosis apparatus with a bearing mechanism for a treatment and/or examination subject comprising a bearing plate for the treatment and/or examination subject that is adjustable at a base, with the bearing plate (1) being seated such that it is height-adjustable and adjustable around three spatial axes (3, 4, 5) and being seated such that it is displaceable in the longitudinal and/or transverse direction by means of a drive device (6), with the bearing plate (1) being seated such that it is pivotable around a longitudinal axis (5) and around a transverse axis (4), **characterised in that** the bearing plate is coupled to the drive device in a radiation-transparent manner in at least one region in order to produce fluoroscopic exposures such that the drive device can optionally be completely disconnected given a horizontal bearing plate, such that a floating bearing of the bearing plate is then established, with the drive device being automatically coupled for locking purposes whenever the bearing plate is tilted or canted, and with the drive device for the pivot around the transverse axis (4) and/or the longitudinal axis (5) being coupled to the lifting drive for the height adjustment and to the drive device (6) for the displacement of the bearing plate (1) such that the bearing plate (1) can be pivoted around a predetermined isocentre 7, and that an exposure unit comprising a radiation transmitter and a radiation receiver can be moved from a parked position, wherein free access to the bearing device is possible, into an exposure position for producing radiation exposures.

2. Operation-diagnosis apparatus according to claim 1, **characterised in that** movements of the bearing plate (1) around one of the rotational axis (3, 4, 5) are only possible when the drive device (6) for the longitudinal and transverse displacement is cut in.

3. Operation-diagnosis apparatus according to claim 1 or 2, **characterised in that** the bearing plate (1) is interchangeably arranged.

4. Operation-diagnosis apparatus according to claim 3, **characterised in that** the bearing plate (1) is optionally fashioned as an angiography bearing plate, an OP bearing plate with one or a plurality of kinks or as a CT bearing plate.

5. Operation-diagnosis apparatus according to claim 3 or 4, comprising a set of interchangeable, different bearing plates (1).

6. Operation-diagnosis apparatus according to claim 5, with the set comprising an angiography bearing plate, an OP bearing plate and/or a CT bearing plate.

## Revendications

1. Unité de diagnostic et d'opération comportant un dispositif de support d'un objet à traiter et/ou analyser, laquelle unité comporte une plaque de support réglable au niveau d'un socle et destinée à l'objet à traiter et/ou analyser, la plaque de support (1) étant montée de façon à être réglable en hauteur et déplaçable autour de trois axes (3, 4, 5) de l'espace, et étant mobile en translation dans une direction longitudinale et/ou transversale au moyen d'un dispositif d'entraînement (6), la plaque de support (1) étant montée de façon à pouvoir pivoter autour d'un axe longitudinal (5) et d'un axe transversal (4), **caractérisée en ce que** la plaque de support est couplée de façon transparente aux rayonnements au dispositif d'entraînement, au moins dans une zone destinée à effectuer des radiographies, de sorte que le dispositif d'entraînement peut être à volonté totalement déconnecté lorsque la plaque de support est en position horizontale de façon à conférer à la plaque de support un montage flottant, le dispositif d'entraînement étant couplé automatiquement lorsque la plaque de support est inclinée ou basculée afin de la bloquer et le dispositif d'entraînement pour le pivotement autour de l'axe transversal (4) et/ou de l'axe longitudinal (5) étant couplé à l'entraînement de levage pour le réglage en hauteur et au dispositif d'entraînement (6) pour le déplacement en translation de la plaque de support (1) de sorte que la plaque de support (1) est apte à pivoter autour d'un isocentre prescriptible (7) et **en ce qu'**une unité de radiographie constituée d'un émetteur de rayonnement et d'un récepteur de rayonnement peut être amenée d'une position de repos, dans laquelle on peut accéder librement au dispositif de support, dans une position d'enregistrement pour faire une radiographie.

2. Unité de diagnostic et d'opération selon la revendication 1, **caractérisée en ce que** la plaque de support (1) est apte à effectuer des mouvements autour de l'un des trois axes de rotation (3, 4, 5) seulement lorsque le dispositif d'entraînement (6) pour le déplacement longitudinal et transversal est connecté.

3. Unité de diagnostic et d'opération selon la revendication 1 ou 2, **caractérisée en ce que** la plaque de support (1) est disposée de façon à être remplaçable.

4. Unité de diagnostic et d'opération selon la revendication 3, **caractérisée en ce que** la plaque de support (1) est conformée au choix en plaque de support pour angiographie (1), en plaque de support opératoire comportant un ou plusieurs coudes ou en plaque de support pour tomographie informatisée.

5. Unité de diagnostic et d'opération selon la revendication 3 ou 4, comportant un ensemble de différentes plaques de support remplaçables (1).

6. Unité de diagnostic et d'opération selon la revendication 5, dans laquelle l'ensemble comporte une plaque de support pour angiographie, une plaque de support opératoire et/ou une plaque de support pour tomographie informatisée.
